(19) **Europäisches Patentamt**

**European Patent Office**

**Office européen des brevets**

(11) **EP 0 723 439 B1**

(12) **EUROPÄISCHE PATENTSCHRIFT**

(45) Veröffentlichungstag und Bekanntmachung des Hinweises auf die Patenterteilung:
**13.09.2000 Patentblatt 2000/37**

(51) Int. Cl.[7]: **A61K 31/00**, A61K 31/565, A61K 31/135, A61K 31/40, A61K 31/38

(21) Anmeldenummer: **94929553.9**

(22) Anmeldetag: **17.10.1994**

(86) Internationale Anmeldenummer:
**PCT/EP94/03408**

(87) Internationale Veröffentlichungsnummer:
**WO 95/11013 (27.04.1995 Gazette 1995/18)**

(54) **KOMBINATION VON PROGESTERONANTAGONISTEN UND ANTIÖSTROGENEN MIT PARTIALER AGONISTISCHER WIRKUNG FÜR DIE HORMONSUBSTITUTIONS-THERAPIE FÜR PERI- UND POSTMENOPAUSALE FRAUEN**

COMBINATION OF PROGESTERONE ANTAGONISTS AND ANTI-OESTROGENS WITH PARTIAL AGONISTIC ACTION FOR USE IN HORMONE-REPLACEMENT THERAPY FOR PERI- AND POST-MENOPAUSAL WOMEN

COMBINAISON D'ANTAGONISTES DE LA PROGESTERONE ET D'ANTI- ESTROGENES A ACTION PARTIELLEMENT AGONISTE POUR L'HORMONOTHERAPIE SUBSTITUTIVE DES FEMMES PERI ET POSTMENOPAUSEES

(84) Benannte Vertragsstaaten:
**AT BE CH DE DK ES FR GB GR IE IT LI LU MC NL PT SE**

(30) Priorität: **17.10.1993 DE 4335876**

(43) Veröffentlichungstag der Anmeldung:
**31.07.1996 Patentblatt 1996/31**

(73) Patentinhaber:
**SCHERING AKTIENGESELLSCHAFT**
**13353 Berlin (DE)**

(72) Erfinder:
• **CHWALISZ, Kristof**
  **D-13503 Berlin (DE)**
• **Stöckemann, Klaus**
  **D-12161 Berlin (DE)**

(56) Entgegenhaltungen:
**EP-A- 0 178 862        EP-A- 0 310 541**
**WO-A-93/17686**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft die Verwendung mindestens einer Verbindung mit prosgesteronantagonistischer (PA) sowie mindestens einer Verbindung mit antiöstrogener (AÖ) bei gleichzeitiger partialer agonistischer Wirkung für die Herstellung von Arzneimitteln für die Hormonsubstitutions-Therapie für peri- und postmenopausale Frauen.

Mit Eintritt der Menopause (Klimakterium) bei Frauen treten aufgrund der veränderten Hormonproduktion sogenannte klimakterische Beschwerden auf. Durch die verminderte Östrogenproduktion steigt gleichzeitig das Osteoporose-Risiko (Verminderung des Knochengewebes bei erhaltener Knochenstruktur durch gesteigerten Knochenabbau und/oder verminderten -anbau); ebenso wird bei postmenopausalen Frauen eine gegenüber prämenopausalen Frauen deutlich erhöhte Herzinfarktsrate sowie ein erhöhtes Auftreten anderer kardiovaskulärer Erkrankungen beobachtet, was ebenfalls auf die verminderte Östrogenproduktion zurückgeführt wird.

Die Hormonsubstitutions-Therapie (hormon replacement therapy = HRT) mit Östrogenen oder mit einer Östrogen/Gestagen-Kombination ist bisher die gängige Methode, um die mit der Menopause verbundenen Symptome zu therapieren (Ernster VL et al. (1988): Benefits and risks of menopausal estrogen and/or progestin hormone use; Prev. Med. 17:201 - 223).

Das Östrogen übt eine protektive Wirkung auf das kardiovaskuläre System, auf die Knochen (Verminderung des Osteoporose-Risikos) und auf das Zentrale Nervensystem (Vermeidung sog. "hot-flushes") aus. Andererseits führt die chronische Anwendung von Östrogenen in der Hormonersatz-Therapie zur Erhöhung des Riskos der Ausbildung eines Endometriumskarzinoms (Ernster VL et al. (1988): Benefits and risks of menopausal estrogen and/or progestin hormone use; Prev. Med. 17:201 - 223).

Durch die gleichzeitige Verwendung eines Gestagens zur Hormonsubstitutions-Therapie wird zwar der stimulierende Effekt des Östrogens auf das Endometrium unterbunden (Gibbson WE, 1986, Biochemical and histologic effects of sequential estrogen/progestin therapy on the endometrium of postmenopausal women; Am. J. Obstet. Gynecol: 154:46 - 61) andererseits können aber bei der kombinierten Therapie mit einem Östrogen und Gestagen die protektiven Effekte der östrogenen Komponente hinsichtlich der Plasmalipide zumindest abgeschwächt werden (Lobo R. (1992): The role of progestins in hormone replacement therapy; Am. J. Obstet. Gynecol. 166: 1997 - 2004).

Außerdem treten unter einer Östrogen/Gestagenbehandlung aufgrund der im Vergleich zu einem oralen Kontrazeptivum verringerten Hormondosierung unerwünschte Zwischenblutungen auf (Hillard TC et al. (1992): Continuous combined conjugated equine estrogen-progestagen therapy: Efects of medroxyprogesterone acetate and norethindrone acetate on bleeding patterns and endometrial histologic diagnosis; A. J. Obstet. Gynecol. 167:1 -7).

Schließlich zeigen neuere Befunde, daß manche Gestagene das Risiko der Ausbildung einer Brustkrebserkrankung erhöhen (Staffa JA et al. (1992): Progestins and breast cancer: an epidemiologic review; 57: 473 - 491); King RJB (1991): A discussion of the roles of estrogen and progestin in human mammary carcinogenesisi; J. Ster. Biochem. Molec. Bio. 39: 8111 - 8118).

Zusammenfassend ergibt sich das Bild, daß die bekannten Östrogen-mono- sowie Östrogen/Gestagenkombinationstherapien keine befriedigenden Möglichkeiten zur Behandlung der mit der Menopause verbundenen Symptome darstellen.

Kürzlich ist auch die Verwendung "echter" Antiöstrogene zur Herstellung von Arzneimitteln für die Hormonersatztherapie (HRT) vorgeschlagen worden (EP-A-0 178 862). Unter "echten" Antiöstrogenen sind gemäß EP-A-0 178 862 beispielsweise Tamoxifen, Nafoxiden, MER-25 gemeint, also solche Antiöstrogene, die rezeptorvermittelt wirken und die gleichzeitig noch eine östrogene (agonistische) Partialwirkung besitzen.

Nachteilig bei einem derartigen, ein "echtes" Antiöstrogen mit partialer östrogener Wirkung enthaltenden Arzneimittel ist, daß, bedingt durch die chronische östrogene Stimulation des Endometriums, wie bei der Anwendung von Östrogenen, ein erhöhtes Risiko der Entstehung eines Endometriumskarzinoms besteht (Fornander T et al. (1989): Adjuvant tamoxifen in early breast cancer: occurence of new primary cancers; Lancet 21: 117 - 119).

Andererseits zeigen sich durch die östrogene Partialwirkung von Tamoxifen positive Effekte auf die Knochen; Tamoxifen scheint bei Frauen den Abbau der Knochenmasse teilweise zu verhindern (Love RR et al. (1992): Effects of tamoxifen on bone mineral density in postmenopausal women with breast cancer; N. Engl. J. Med. 26:852 - 856).

Außerdem haben Untersuchungen mit Tamoxifen gezeigt, daß dessen antiöstrogene Komponente für die Wachstumshemmung beim Einsatz in der Therapie des Mammakarzinoms bei postmenopausalen Frauen verantwortlich ist (Buckley MMT et al. (1989); Tamoxifen: A reappraisal of its pharmocodynamic and pharmacokinetic properties and therapeutic use; Drugs 37: 451 - 490).

Demnach ist die erforderliche chronische Anwendung eines Antiöstrogens mit agonistischer Partialwirkung in der Hormonsubstitutions-Therapie als bedenklich anzusehen, da eine Stimulation des Endometriums die Entstehung eines Endometriumkarzinoms begünstigen kann.

Aufgabe der vorliegenden Erfindung ist es deshalb, ein Arzneimittel für die Hormonsubstitutions-Therapie (HRT) bereitzustellen, welches die unerwünschten Wirkungen bei einer chronischen Monotherapie mit Antiöstrogenen mit partialer agonistischer Wirkung (Stimulation des Endometriums) verhindert, gleichzeitig aber die protektive Wirkung auf die Knochen und das kardiovaskuläre System (aufgrund der agonistischen Wirkung) sowie die Mamma (antagonistische Wirkung) unbeeinflußt läßt, bzw. welches die protektiven Effekte sogar verstärkt.

Diese Aufgabe wird durch die vorliegende Erfindung gelöst, und zwar durch die Verwendung mindestens einer Verbindung mit progesteronantagonischer (PA) sowie mindestens einer Verbindung mit antiöstrogeuer (AÖ) bei gleichzeitiger partialer agonistischer Wirkung zur Herstellung eines derartigen Arzneimittels.

Es wurde gefunden, daß bei dem erfindungsgemäß hergestellten Arzneimittel, die Komponente mit progesteronantagonistischer (PA) Wirkung die durch die partiale östrogene Wirkung des Antiöstrogens verursachten Veränderungen (Stimulation des Myo- und Endometriums) lediglich im Uterus inhibieren, überraschenderweise jedoch die anderen, in der Hormonersatz-Therapie überaus erwünschten Effekte (beispielsweise am Knochen und auf das kardiovaskuläre System) erhalten bleiben.

Die aufgefundene, vorteilhafte Wirkung des erfindungsgemäß hergestellten Arzneimittels kommt vermutlich dadurch zustande, daß die partiale östrogene Wirkung des Antiöstrogens (Jordan VC et al. (1979): Effects of oestradiol benzoate, tamoxifen and monohydroxytamoxifen on immature rat uterine progesterone receptor synthesis and endometrial cell division; J. Steroid. Biochem. 11: 285 - 291) durch den antiproliferativen Effekt des kompetitiven Progesteronantagonisten (PA) inhibiert wird (Wolf JP et al. (1989): Noncompetitive antiestrogenic effect of RU 486 in blocking the estrogen-stimulated luteinizing hormone surge and the proliferative action of estradiol on endometrium in castrate monkeys; Fertil. Steril. 52: 1055 - 1060; Chwalisz K et al. (1992): Evaluation of the antiproliferative actions of the progesterone antagonists mifepristone (RU 486) and onapristone (ZK 98 299) on primate endometrium; Society of Gynecologic Investigation, 39th Annual Meeting, San Antonio, Texas, Abstract). Der Progesteronantagonist übt selektiv eine Schutzfunktion auf das Endometium aus.

Es konnte gezeigt werden, daß bei ovarektomierten Ratten (als Tiermodell für die postmenopausale Frau) die durch Östradiol stimulierte Proliferation des Myometriums bzw. Endometriums durch kompetitive Progesteronantagonisten inhibiert wird. Allerdings sind hier vor allem die stromalen bzw. myometrialen Bereiche betroffen, weniger das luminare Epithel. Bei der Kombination eines Antiöstrogens mit partialer östrogener Wirkung (z.B. Tamoxifen) mit einem kompetitiven Progesteronantagonisten (PA) (Onapriston) wurde nun gefunden, daß sowohl die myometrialen, als auch die stromalen und epithelialen Bereiche im Uterus inhibiert werden.

Die erfindugsgemäß hergestellten Arzneimittel sind somit für einen präventiven als auch für einen kurativen Einsatz in der Hormonsubstitutions-Therapie (HRT) geeignet, da durch die östrogene Partialwirkung des Antiöstrogens ein Abbau von Knochenmasse verhindert wird, gleichzeitig die östrogene Komponente protektiv auf das kardiovaskuläre System wirkt und die unerwünschten stimulierenden Effekte auf das Endometrium durch die antiproliferative Wirkung des kompetitiven Progesteronantagonisten, im Sinne einer Schutzfunktion, verbindert wird.

Diese Arzneimittel sind somit für eine Langzeitanwendung in der HRT geeignet und können kontinuierlich oder intermittierend gegeben angewendet werden.

Daß progesteronantagonistisch wirksame Verbindungen in Kombination mit antiöstrogen wirksamen Verbindungen zur Herstellung von Arzneimitteln zur Geburtseinleitung, zum Schwangerschaftsabbruch sowie zur Behandlung gynäkologischer Störungen (Dysmennorrhoe und Endometriose) verwendet werden können, ist bereits aus der EP-A-0 310 541 bekannt.

Das Gewichtsverhältnis beider Komponenten in dem neuen Arzneimittel kann dabei in weiten Grenzen variiert werden. So können sowohl gleiche Mengen PA und AÖ als auch ein Überschuß einer der beiden Komponenten eingesetzt werden. PA und AÖ werden gemeinsam getrennt, gleichzeitig und/oder zeitlich abgestuft (sequential), in einem Gewichtsverhältnis von im wesentlichen 50:1 bis 1:50, vorzugsweise 25:1 bis 1:25, und insbesondere 10:1 bis 1:10 verwendet. Die gleichzeitige Gabe ist bevorzugt. Im Falle der sequentiellen Gabe kann die als zweite gegebene Verbindung zu jeder Zeit nach Gabe der zuerst applizierten Verbindung gegeben werden, solange sie noch in der Patientin gleichzeitig mit einer wirksamen Menge der zuerst applizierten Verbindung bioverfügbar wird. Beispielsweise kann das AÖ ab dem 2. Tag nach der Applikation von PA gegeben werden, wobei ab dem 3. Tag dann sowohl PA als auch AÖ appliziert werden können.

Vorzugsweise können PA und AÖ kombiniert in einer Dosiseinheit appliziert werden.

Im allgemeinen ist eine einmalige tägliche Applikation der beiden Komponenten ausreichend.

Die Dauer der Behandlung mit dem erfindungsgemäßen Arzneimittel ist zeitlich nicht begrenzt; eine chronische Behandlung kann auch intermittierend durchgeführt werden, d.h. an einen längeren Zeitraum, in dem die Komponenten appliziert werden, schließt sich jeweils eine kürzere Einnahmepause an; beispielsweise wird 3 bis 6 Monate lang behandelt woran sich eine ungefähr 2 monatige Einnahmepause anschließt.

Als kompetitive Progesteronantagonisten kommen alle Verbindungen in Frage, die die Wirkung des Progesterons am Gestagenrezeptor (Progesteronrezeptor) kompetitiv blockieren und dabei keine eigene gestagene Aktivität zeigen; diese Blockade kann durch die verabreichte Substanz selbst oder durch deren Metaboliten bewirkt werden. Beispielsweise kommen folgende Steroide in Frage:

11β-[(4-N,N-Dimethylamino)-phenyl]-17β-hydroxy-17α-propinyl-4,9(10)-estradien-3-on (RU-38486),
11β-[(4-N,N-Dimethylamino)-phenyl]-17β-hydroxy-18-methyl-17α-propinyl-4,9(10)-estradien-3-on und
11β-[(4-N,N-Dimethylamino)-phenyl]-17aβ-hydroxy-17aα-propinyl-D-homo-4,9(10),16-estratrien-3-on (alle EP-A-0 057 115), ferner

11β-p-Methoxyphenyl-17β-hydroxy-17α-ethinyl-4,9(10)-estradien-3-on (Steroids 37 (1981), 361-382), 11β-(4-Acetylphenyl)-17β-hydroxy-17α-(prop-1-inyl)-4,9(10)-estradien-3-on (EP-A 0 190 759), sowie die in der EP-A 0 277 676 beschriebenen 11β-Aryl-14β-estradiene und -triene, die 19,11β-überbrückten Steroide, die Gegenstand der EP-A-0 283 428 sind, die aus der EP-A-0 289 073 hervorgehenden 11β-Aryl-6-alkyl (bzw. 6-Alkenyl oder 6-alkinyl)-estradiene und - pregnadiene und die aus der EP-A-0 321 010 bekannten 11β-Aryl-7-methyl (bzw. 7-ethyl)-estradiene sowie die 108-H-Steroide der EP-A-0 404 283, beispielsweise (Z)-11β-[4-(Dimethylamino)phenyl]-17α-(3-hydroxyprop-1-enyl)-estr-4-en-17β-ol.

Weiterhin seien als typische Vertreter erfindungsgemäß zu verwendender, kompetitiver Progesteronantagonisten beispielsweise genannt:

11β-(4-Dimethylamino)-17α-hydroxy-17β-(3-hydroxy-propyl)-13α-methyl-4,9-gonadien-3-on (EP-A-0 129 499);
11β-(4-Acetylphenyl)-17β-hydroxy-17α-(3-hydroxyprop-1(Z)-enyl)-4,9(10)-estradien-3-on (EP-A-0 190 759);
11β,19-[4-(Cyanphenyl)-o-phenylen]-17β-hydroxy-17α-(3-hydroxyprop-1(Z)-enyl)-4-androsten-3-on und

11β,19-[4-(3-Pyridinyl)-o-phenylen]-17β-hydroxy-17α-(3-hydroxyprop-1(Z)-enyl)-4-androsten-3-on  (beide  EP-A-0 283 428).

Die Aufzählung der PA ist nicht abschließend; auch andere in den genannten Veröffentlichungen beschriebene kompetitive Progesteronantagonisten sowie solche aus hier nicht genannten Veröffentlichungen sind geeignet.
Die kompetitiven Progesteronantagonisten können zum Beispiel lokal, topisch, enteral, transdermal oder parenteral appliziert werden.
Für die bevorzugte orale Applikation kommen insbesondere Tabletten, Dragèes, Kapseln, Pillen, Suspensionen oder Lösungen in Frage, die in üblicher Weise mit den in der Galenik gebräuchlichen Zusätzen und Trägersubstanzen hergestellt werden können. Für die lokale oder topische Anwendung kommen beispielsweise Vaginalzäpfchen, Vaginalgels, Implantate, Vaginalringe, intrauterine Freisetzungssysteme (IUDs) oder transdermale Systeme wie Hautpflaster in Frage.
Eine Dosierungseinheit enthält etwa 0,25 bis 50 mg 11β-[(4-N,N-Dimethylamino)-phenyl]-17α-hydroxy-17β-(3-hydroxypropyl)-13α-methyl-4,9(10)-gonadien-3-on oder eine biologisch äquivalente Menge eines anderen kompetitiven Progesteronantagonisten.
Erfolgt die Applikation des erfindungsgemäß hergestellten pharmazeutischen Mittels durch ein Implantat, einen Vaginalring, ein IUD oder ein transdermales System, so müssen diese Applikationssysteme derart ausgebildet sein, daß die durch sie täglich freigesetzte Dosis des kompetitiven Progesteronantagonisten in diesem Bereich von 0,25 bis 50 mg liegt.
Als Antiöstrogene mit partialer agonistischer (östrogener) Wirkung kommen alle gebräuchlichen derartigen Antiöstrogene in Betracht. Sie können etwa in gleichen Mengen eingesetzt werden wie die bereits im Handel befindlichen Antiöstrogene, das heißt die tägliche Dosis beträgt etwa 5-100 mg für Tamoxifen oder biologisch äquivalente Mengen eines anderen Antiöstrogens. Die tägliche Dosis ist in jedem Fall so zu wählen, daß am Endometrium ein atrophischer Zustand entsteht, die Östrogeneffekte auf die Knochen und das kardiovaskuläre System jedoch erhalten bleiben (Substitution). Aufgrund seiner hohen Östrogenrezeptor-Konzentration spricht das Endometrium sensibler auf Östrogene bzw. Antiöstrogene als andere Zielorgane an. Als Antiöstrogene seien beispielsweise genannt:

Tamoxifen  = (Z)-2-[p-(1,2-Diphenyl-1-butenyl)-phenoxy]-N,N-dimethyl-äthylamin,
Nafoxidin  = 1-2-[4-(6-Methoxy-2-phenyl-3,4-dihydro-1-naphthyl)-phenoxy]-äthylpyrrolidin, Hydrochlorid,
Mer 25  = 1-[p-(2-Diäthylaminoäthoxy)-phenyl]-2-(p-methoxyphenyl)-1-phenyläthanol
Raloxifen  = 6-Hydroxy-2-(p-hydroxyphenyl)benzo[b]thien-3-yl-p-(2-piperidinoethoxy)phenylketon, Hydrochlorid

Progesteronantagonistisch- und antiöstrogen wirksame Verbindungen können z. B. lokal, topisch, enteral oder parenteral appliziert werden.
Für die bevorzugte enterale Applikation kommen insbesondere Tabletten, Dragèes, Kapseln, Pillen, Suspensionen oder Lösungen infrage, die in üblicher Weise mit den in der Galenik üblichen Zusätzen und Trägersubstanzen hergestellt werden können. Für die lokale oder topische Anwendung kommen beispielsweise Vaginalzäpfchen oder transdermale Systeme wie Hautpflaster infrage.

Eine AÖ-Dosiseinheit enthält 1-100 mg Tamoxifen oder eine biologisch äquivalente Menge einer anderen antiöstrogen wirksamen Verbindung.

Die nachfolgenden Beispiele dienen der näheren Erläuterung der Erfindung:

**Beispiel 1**

| 10,0 mg | 11β-[(4-N,N-Dimethylamino)-phenyl]-17α-hydroxy-17β-(3-hydroxypropyl)-13α-methyl-4,9-gonadien-3-on |
|---|---|
| 140,5 mg | Laktose |
| 69,5 mg | Maisstärke |
| 2,5 mg | Poly-N-vinylpyrrolidon |
| 2,0 mg | Aerosil |
| 0.5 mg | Magnesiumstearat |
| 225,0 mg | Gesamtgewicht der Tablette |

**Beispiel 2**

| 20,0 mg | Tamoxifen (Antiestrogen mit agonistischer Partialwirkung) |
|---|---|
| 50,0 mg | 11β-[(4-N,N-Dimethylamino)-phenyl]-17α-hydroxy-17β-(3-hydroxypropyl)-13α-methyl-4,9-gonadien-3-on |
| 105,0 mg | Laktose |
| 40,0 mg | Maisstärke |
| 2,5 mg | Poly-N-vinylpyrrolidon 25 |
| 2,0 mg | Aerosil |
| 0,5 mg | Magnesiumstearat |
| 220,0 mg | Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden. |

**Beispiel 3**

| 10,0 mg | Raloxifen |
|---|---|
| 30,0 mg | 11β-[(4-N,N-Dimethylamino)-phenyl]-17α-hydroxy-17β-(3-hydroxypropyl)-13α-methyl-4,9-gonadien-3-on |
| 125,0 mg | Laktose |
| 50,0 mg | Maisstärke |
| 2,5 mg | Poly-N-vinylpyrrolidon 25 |
| 2,0 mg | Aerosil |
| 0.5 mg | Magnesiumstearat |
| 220,0 mg | Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden. |

**Beispiel 4**

**Zusammensetzung einer öligen Lösung:**

| 100,0 mg | Tamoxifen |
|---|---|
| 343,4 mg | Rizinusöl |
| 608.6 mg | Benzylbenzoat |
| 1052,0 mg | =1 ml |

Die Lösung wird in eine Ampulle gefüllt.

*Pharmakologische Beobachtungen*

Die Versuche wurden an ovarektomierten (ovx) Ratten (n = 10 Tiere/Gruppe) durchgeführt (Gr. 1 bis Gr. 6). Die ovarektomierten Tiere wurden über 3 bis 8 Tage mit Östradiol + Onapriston (0,3 μg + 10,0 mg/Tag/Tier) bzw. mit dem Antiöstrogen Tamoxifen (0,2 mg/Tag/Tier) + Onapriston (10,0 mg/Tag/Tier) s.c. behandelt. Am Ende des Versuches wurden die Uteri gewogen und eine routinemäßige histologische Begutachtung vorgenommen.

*Ergebnisse*

Die Behandlung mit Östradiol alleine führt zu einer Stimulation des Myometriums, des stromalen und epithelialen Gewebes im Uterus (Gr. 2). Durch gleichzeitige Gabe des kompetitiven Progesteronantagonisten Onapriston werden die Effekte auf das Myometrium bzw. Stroma größtenteils, die auf das Epithel teilweise inhibiert (Gr. 4). Onapriston allein hat keinen Einfluß auf das uterine Gewebe (Gr.3). Die Behandlung von ovarektomierten Ratten mit Tamoxifen alleine (Antiöstrogen mit partialer östrogener Wirkung) führt wie die Behandlung mit Östradiol zu einer Stimulation verschiedener uteriner Gewebskomponenten und des Uterusgewichtes (Gr. 5). Durch Kombination mit einem kompetitiven Progesteronantagonisten (Onapriston) kann der stimulierende Effekt von Tamoxifen auf das Endometrium (Stroma und Epithel) aufgehoben werden (Gr. 6).

Tabelle 1

| Morphologische Veränderungen im Uterus | | | | | |
|---|---|---|---|---|---|
| Gr. 1 | Substanz | Myometrium | Endometrium | | Uterusgewichte |
| | | | Stroma | Epithel | |
| 1 | ovx + Vehikel | - | - | - | - |
| 2 | ovx + Östradiol | +++ | +++ | +++ | +++ |
| 3 | ovx + Onapriston | - | - | - | - |
| 4 | ovx + Östradiol + Onapriston | + | + | ++ | + |
| 5 | ovx + Tamoxifen | ++ | ++ | ++ | ++ |
| 6 | ovx + Tamoxifen + Onapriston | + | - | - | + |
| + = Stimulation  - = Inhibition | | | | | |

**Patentansprüche**

**1.** Verwendung mindestens einer Verbindung mit progesteronantagonistischer (PA) sowie mindestens einer Verbindung mit antiöstrogener (AÖ) bei gleichzeitiger partialer agonistischer Wirkung für die Herstellung von Arzneimitteln für die Hormonsubstitutions-Therapie (HRT) für peri- und postmenopausale Frauen.

**2.** Verwendung von 11β-[(4-N,N-Dimethylamino)-phenyl]-17β-hydroxy-17α-propinyl-4,9(10)-estradien-3-on oder 11β-(4-Dimethylamino)-17α-hydroxy-17β-(3-hydroxypropyl)-13α-methyl-4,9-gonadien-3-on als Verbindung mit progesteronantagonistischer (PA) Wirkung nach Anspruch 1.

**3.** Verwendung von (Z)-2-[p-(1,2-Diphenyl-1-butenyl)-phenoxy]-N,N-dimethyläthylamin, 1-2-[4-(6-Methoxy-2-phenyl-3,4-dihydro-1-naphthyl)-phenoxy]-äthylpyrrolidin, Hydrochlorid, 1-[p-(2-Diäthylaminoäthoxy)-phenyl]-2-(p-methoxyphenyl)-1-phenyläthanol, oder 6-Hydroxy-2-(p-hydroxyphenyl)benzo[b]thien-3-yl-p-(2-piperidinoethoxy)phenylketon, Hydrochlorid, als Verbindung mit antiöstrogener (AÖ) Wirkung nach Anspruch 1.

**4.** Verwendung von 11β-(4-Dimethylamino)-17α-hydroxy-17β-(3-hydroxy-propyl)-13α-methyl-4,9-gonadien-3-on (PA) und (Z)-2-[p-(1,2-Diphenyl-1-butenyl)-phenoxy]-N,N-dimethyl-äthylamin (AÖ) nach Anspruch 1.

**Claims**

**1.** Use of at least one compound having progesterone-antagonistic (PA) action and at least one compound having antioestrogenic (AO) action with simultaneous partial agonistic action for the production of medicaments for hormone replacement therapy (HRT) for peri- and post-menopausal women.

2. Use of 11β-[(4-N,N-dimethylamino)phenyl]-17β-hydroxy-17α-propynyl-4,9(10)-oestradien-3-one or 11β- (4-dimethylamino)-17α-hydroxy-17β-(3-hydroxypropyl)-13α-methyl-4,9-gonadien-3-one as a compound having progesterone-antagonistic (PA) action according to Claim 1.

3. Use of (Z)-2-[p-(1,2-diphenyl-1-butenyl)-phenoxy]-N,N-dimethylethylamine, 1-2- [4- (6-methoxy-2-phenyl-3,4-dihydro-1-naphthyl)phenoxy]ethylpyrrolidine, hydrochloride, 1- [p- (2-diethylaminoethoxy) phenyl]-2-(p-methoxyphenyl)-1-phenylethanol or 6-hydroxy-2-(p-hydroxyphenyl)benzo [b] thien-3-yl p- (2-piperidinoethoxy) phenyl ketone, hydrochloride, as a compound having antioestrogenic (AO) action according to Claim 1.

4. Use of 11β-(4-dimethylamino)phenyl]-17α-hydroxy-17β-(3-hydroxypropyl)-13α-methyl-4,9-gonadien-3-one (PA) or (Z)-2- [p-(1,2-diphenyl-1-butenyl)phenoxy]-N,N-dimethylethylamine (AO) according to Claim 1.

**Revendications**

1. Utilisation d'au moins un composé à action antagoniste de la progestérone (PA) ainsi que d'au moins un composé à action antioestrogène (AO) à action agoniste partielle simultanée pour la préparation de médicaments pour l'hormonothérapie substitutive (HRT) pour les femmes péri- et postménopausées.

2. Utilisation de 11β-[(4-N,N-diméthylamino)-phényl]-17β-hydroxy-17α-propynyl-4,9(10)-estradién-3-one ou de 11β-(4-diméthylamino)-17α-hydroxy-17β-(3-hydroxypropyl) -13α-méthyl-4,9-gonadién-3-one comme composé à action antagoniste de la progestérone (PA) suivant la revendication 1.

3. Utilisation de (Z)-2-[p-(1,2-diphényl-1-butényl) phénoxy]-N, N-diméthyléthylamine, de chlorhydrate de 1-2-[4- (6-méthoxy-2-phényl-3,4-dihydro-1-naphtyl) phénoxy]éthylpyrrolidine, de 1-[p- (2-diéthylaminoéthoxy)phényl]-2-(p-méthoxyphényl)-1-phényléthanol, ou de chlorhydrate de 6-hydroxy-2-(p-hydroxyphényl)benzo[b]thién-3-yl-p-2-pipéridinoéthoxy)phényl-cétone, comme composé à action antioestrogène (AO) suivant la revendication 1.

4. Utilisation de 11β-(4-diméthylamino)-17α-hydroxy-17β-(3-hydroxy-propyl)-13α-méthyl-4,9-gonadién-3-one (PA) et de (Z) -2-[p- (1, 2-diphényl-1-butényl)phénoxy]-N,N-diméthyléthylamine (AO) suivant la revendication 1.